# EUROPEAN PATENT SPECIFICATION

(11) **EP 1 599 740 B1**
(45) Date of publication and mention of the grant of the patent: **18.02.2009**
(21) Application number: 04714611.3
(22) Date of filing: 25.02.2004
(51) Int. Cl.: G01R 33/28

(54) **MRI/NMR COMPATIBLE HYPERPOLARIZED GAS DELIVERY VALVES FOR VENTILATORS AND ASSOCIATED GAS DELIVERY METHODS**
FÜR MRI/NMR GEEIGNETES VENTIL FÜR DIE GASZUFÜHRUNG EINES VENTILATORS UND DAMIT VERBUNDENE VERFAHREN DER GASZUFÜHRUNG
SOUPAPES DE DISTRIBUTION DE GAZ HYPERPOLARISES COMPATIBLES IRM/RMN DESTINEES A DES VENTILATEURS ET PROCEDES DE DISTRIBUTION DE GAZ ASSOCIES

(30) Priority: 26.02.2003 US 450209 P
(43) Date of publication of application: 30.11.2005
(73) Proprietor: Medi-Physics Inc., Princeton, NJ 08540 (US)
(72) Inventor: BOLAM, Kenneth, Raleigh, NC 27614 (US); DAVIDSON, James, US Flagstaff, AZ 86004 (US); BORGEN, James, Durham, NC 27713 (US)
(74) Representative: Canning, Lewis R.
(86) International application number: PCT/US2004/005567
(87) International publication number: WO 2004/077085

(56) References cited:
- WO-A-99/25243
- HEDLUND L W ET AL: "MR-COMPATIBLE VENTILATOR FOR SMALL ANIMALS: COMPUTER-CONTROLLED VENTILATION FOR PROTIN AND NOBLE GAS IMAGING" MAGNETIC RESONANCE IMAGING, vol. 18, 2000, pages 753-759, XP001029472 ISSN: 0730-725X cited in the application
- L.W. HEDLUND ET AL.: 'Mixing oxygen with hyperpolarized (3)He for small-animal lung studies.' NMR IN BIOMEDICINE vol. 13, 2000, pages 202 - 206, XP008033326

## Description

### Field of the Invention

The present invention relates to the delivery of polarized noble gases to subjects for evaluations using NMR spectroscopy and/or magnetic resonance imaging ("MRI").

### Background of the Invention

Polarized inert noble gases can produce improved MRI images of certain areas and regions of the body that have heretofore produced less than satisfactory images in this modality. Polarized helium-3 ("³He") and xenon-129 ("¹²⁹Xe") have been found to be particularly suited for this purpose. Unfortunately, the polarized state of the gases is sensitive to handling and environmental conditions and can, undesirably, decay from the polarized state relatively quickly.

Hyperpolarizers are used to produce and accumulate polarized noble gases. Hyperpolarizers artificially enhance the polarization of certain noble gas nuclei (such as ¹²⁹Xe or³He) over the natural or equilibrium levels, *i.e.,* the Boltzmann polarization. Such an increase is desirable because it enhances and increases the MRI signal intensity, allowing physicians to obtain better images of the substance in the body. *See* U.S. Patent Nos. 5,545,396; 5,642,625; 5,809,801; 6,079,213, and 6,295,834.

In order to produce the hyperpolarized gas, the noble gas is typically blended with optically pumped alkali metal vapors such as rubidium ("Rb"). These optically pumped metal vapors collide with the nuclei of the noble gas and hyperpolarize the noble gas through a phenomenon known as "spin-exchange-" The "optical pumping" of the alkali metal vapor is produced by irradiating the alkali-metal vapor with circularly polarized light at the wavelength of the first principal resonance for the alkali metal (e.g., 795 nm for Rb). Generally stated, the ground state atoms become excited, then subsequently decay back to the ground state. Under a modest magnetic field 1mT; (10 Gauss), the cycling of atoms between the ground and excited states can yield nearly 100% polarization of the atoms in a few microseconds. This polarization is generally carried by the lone valence electron characteristics of the alkali metal. In the presence of non-zero nuclear spin noble gases, the alkali-metal vapor atoms can collide with the noble gas atoms in a manner in which the polarization of the valence electrons is transferred to the noble-gas nuclei through a mutual spin flip "spin-exchange." After the spin-exchange has been completed, the hyperpolarized gas is typically separated from the alkali metal prior to administration to a patient (to form a non-toxic pharmaceutically acceptable product). Unfortunately, during production and/or during and after collection, the hyperpolarized gas can deteriorate or decay relatively quickly (lose its hyperpolarized state) and therefore must be handled, collected, transported, and stored carefully.

In the past, several researchers have used hyperpolarized gas compatible ventilators for delivering polarized gas to subjects to image hyperpolarized noble gases such as helium and xenon. For example, Hedlund et al., in MR-compatible ventilator for small animals; computer controlled ventilation for proton and noble gas imaging, 18 Magnetic Resonance Imaging, pp. 753-759 (2000), state that ventilators have been in routine use in their laboratory for a number of years. *See also,* Hedlund et al., Three-dimensional MR microscopy of pulmonary dynamics, Society of Magnetic Resonance (New York, NY, 1996); and a poster presented by Hedlund et al. at the Amer. Thoracic Society 1998 International Meeting (Chicago, 1998), entitled *MRI of pulmonary airways with hyperpolarized helium; a computer-controlled ventilator for imaging synchronous gas delivery in animal studies* (describing ventilator technology). In addition, Black and co-workers have used a hyperpolarized gas-compatible ventilator to generate what is believed to be the first ever *in vivo* images of hyperpolarized ³He in guinea pig lungs. *See* Black et al., In vivo He-3 MR images of guinea pig lungs, Radiology, 199(3), pp. 867-870 (1996). Unfortunately, the valves used to control the delivery of the polarized gases have, in the past, been limited by one or more of slow response times, relatively limited flow rates, and/or limited respiration rates (about 120 breaths per minute (BPM)).

Despite the foregoing, there remains a need to provide improved valve configurations for ventilation delivery of hyperpolarized gas.

### Summary of the Invention

The present invention provides improved MRI compatible hyperpolarized gas delivery valves that employ spool configurations.

In particular embodiments, the valve configuration uses multiple spools to controllably sequentially deliver for inhalation, a plurality of gases, such as a hyperpolarized gas and a non-polarized gas to a subject. The multiple-spool valve can be configured to controllably select between three or more different flow paths that allow for inhale, exhale, and breath-hold flow paths as well as delivery of different gas formulations.

In operation, the spool valve configuration can employ a command pilot gas signal with a pressure of between about 200-400 Pa [30-60 psi]. In addition, the spool valves can be configured to react to the pilot pressure in a relatively short response time, such as in under about 50 ms, and typically in about 40ms (which is less than about half the response time of diaphragm type valves) to alter the valve flow path to transmit the desired gas to the subject. In certain embodiments, the valve is configured to operate with a respiration or breath rate (breaths per minute or "BPM") of at least 150 BPM, typically up to at least 180 BPM, and in certain embodiments about 200 BPM or more. Thus, in certain embodiments, the gas delivery valve can provide a fast response time for a high BPM with a low inhale/exhale ratio.

The gas delivery valves using at least one spool to actuate at least one gas flow path in a valve body may be particularly suitable for small animal ventilators used during NMR spectroscopy and/or MRI imaging sessions. The gas delivery valves can provide fast response times, which may be particularly suitable for use in situations where the subject is ventilated at a high BPM breathing rate with a low inhale/exhale ratio (I/E). The I/E ratio is the inspiration time over the expiration time, inspiration is the time during which the gas is provided to the animal (or other subject) while expiration is the time during which the animal (or other subject) is exhaling. Therefore, a high BPM, such as about 180 BPM, means that each breath lasts 333 ms. An I/E of 20/80 means that the device has 67 ms to deliver the desired tidal volume to the animal (or other subject). The speed at which the valve opens can be important, the faster the BPM, the less time the valve has to open and supply the desired amount of gas to the animal (or other subject).

The I/E ratio is typically within about 30/70 to 60/40, but can vary outside of this range, depending on the desired result. The I/E ratio and BPM can be substantially fixed during "normal" (non-imaging) breathing. The BPM and I/E ratio can be adjusted to be different for imaging (hyperpolarized gas) breath runs, but once the image run is over, these operating parameters can return to "normal" breathing parameters.

Also, it is noted that 180 BPM may be an upper range for many small animal species and, in certain embodiments, the ventilator may operate at a lower rate. The operating parameters are animal species/weight driven. For example, mice use a faster BPM than a rabbit. The I/E ratio can change between animals but will typically stay within the range mentioned above.

The gas delivery valve for a ventilator is configured to supply hyperpolarized gas to a subject and includes: (a) a valve body having at least one gas flow path extending between at least one gas inlet port and at least one gas exit port and at least one spool receptacle (or bore), one of the at least one gas inlet ports being in fluid communication with a hyperpolarized gas source; (b) at least one spool member disposed in a respective one of said at least one spool receptacles of said valve body, said spool member configured and sized to cooperate with said valve body to selectively open and close the at least one gas flow path; and (c) at least one pilot fluid port in fluid communication with said spool receptacle. In operation, a pilot fluid command pulse signal having an associated pressure and duration is transmitted into said pilot fluid port forcing said spool to translate an actuation distance in a predetermined direction in said valve body receptacle to thereby open and/or close the at least one gas flow path.

In certain embodiments, the present invention can provide relatively quick actuation of a double acting spool using a single pulse of pilot air to actuate the spool(s) in the valve, allowing the pilot pressure to then be released in between alternating pulses of pilot air to toggle from inhale to exhale. The spool can be actuated during the rising (initial portion) of the pressure response profile or curve.

The foregoing and other objects and aspects of the present invention are explained in detail herein.

### Brief Description of the Drawings

**Figure 1** is a block diagram of operations that can be carried out according to embodiments of the present invention.
**Figures 2A** and **2B** are schematic views of a spool valve configured to operate in response to pilot gas to define a respective one of two selectable gas flow paths according to embodiments of the present invention.
**Figure 3** is a cutaway view of a gas delivery valve with multiple spools according to embodiments of the present invention.
**Figures 4A-4H** are schematic illustrations of different valve positions of a three-spool valve configuration according to embodiments of the present invention.
**Figure 5** depicts three spool components of the present invention.
**Figures 6A-6C** are schematic illustrations of a prior art gas delivery valve using a diaphragm configuration.
**Figure 7** is a graph of pressure as a function of time (ms) illustrating the response of a diaphragm configuration for a prior art gas delivery valve.
**Figure 8** is a graph of pressure versus time (ms) of the response of a toggle spool valve according to embodiments of the present invention.

### Detailed Description of Embodiments of the Invention

The present invention will now be described more fully hereinafter with reference to the accompanying figures, in which preferred embodiments of the invention are shown. This invention, defined in the claims, may, however, be embodied in many different forms and should not be construed as limited to the embodiments set forth herein. Like numbers refer to like elements throughout. In the drawings, layers, regions, or components may be exaggerated for clarity. In the figures, broken lines indicate optional features unless described otherwise.

In the description of the present invention that follows, certain terms may be employed to refer to the positional relationship of certain structures relative to other structures. As used herein the term "forward" and derivatives thereof refer to the general direction the target gas or target gas mixture travels as it moves through the hyperpolarizer system; this term is meant to be synonymous with the term "downstream," which is often used in manufacturing environments to indicate that certain material being acted upon is farther along in the manufacturing process than other material. Conversely, the terms "rearward" and "upstream" and derivatives thereof refer to the directions opposite, respectively, the forward and downstream directions.

Also, as described herein, polarized gases are produced and collected and may, in particular embodiments be frozen, thawed, used alone and/or combined with other constituents, for MRI and/or NMR spectroscopy applications. For ease of description, the term "frozen polarized gas" means that the polarized gas has been frozen into a solid state. The term "liquid polarized gas" means that the polarized gas has been or is being liquefied into a liquid state. Thus, although each term includes the word "gas," this word is used to name and descriptively track the gas that is produced via a hyperpolarizer to obtain a polarized "gas" product. Thus, as used herein, the term "gas" or "target gas" has been used in certain places to descriptively indicate a hyperpolarized noble gas product and may be used with modifiers such as "solid", "frozen", and "liquid" to describe the state or phase of that product. As also used herein, the term "polarized gas" includes at least one polarized gas of interest (such as, but not limited to, ³He and/or ¹²⁹Xe) and may include one or more other constituents such as other carrier, blending or buffer gases. Further, the terms "polarize", "polarizer", "polarized", and the like are used interchangeably with the terms "hyperpolarize", "hyperpolarizer", "hyperpolarized" and the like.

The gas delivery valves contemplated by the present invention can be used for any ventilator systems for target subjects and are hyperpolarized gas-compatible for NMR and/or MRI procedures. These valves have been used in non-hyperpolarised gas-compatible ventilator systems, such as reported by Rosenthal and Li (J.Appl. Physiol. 1993; 83(5): 1768-74), although not specifically for gas delivery. "Subtects", according to the present invention, can be any animal subject, and are preferable mammalian subjects (*e.g*., humans, canines felines, bovines, caprines, ovines, equines, rodents, porcines, and/or lagomorphs). The tcrm"small animals" includes mice, rats, guinea pigs, dogs, cats, monkeys, pigs, and rabbits.

Various techniques have been employed to accumulate and capture polarized gases. For example, U.S. Patent No. 5,642,625 to Cates et al. describes a high volume hyperpolarizer for spin-exchange polarized noble gas and U.S. Patent No. 5,809,801 to Cates et al. describes a cryogenic accumulation for spin-polarized ¹²⁹Xe. As used herein, the terms "hyperpolarize," "polarize," and the like, are used interchangeably and mean to artificially enhance the polarization of certain noble gas nuclei over the natural or equilibrium levels. Such an increase is desirable because it allows stronger imaging signals corresponding to better NMR spectroscopy and/or MRI images of the substance and a targeted area of the body. As is known by those of skill in the art, hyperpolarization can be induced by spin-exchange with an optically pumped alkali-metal vapor or alternatively by metastability exchange. *See* Albert et aL, U.S. Patent No. 5,545,396.

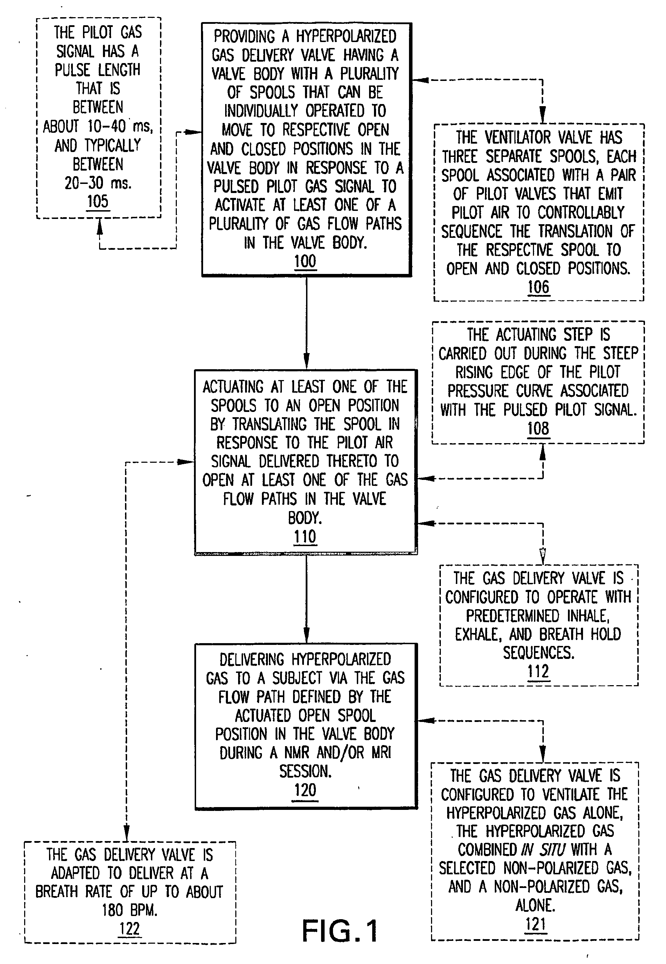

Turning now to **Figure 1**, a hyperpolarized gas delivery valve having a valve body with a plurality of spools and a plurality of selectable gas flow paths is provided (block 100). The spools are disposed in the valve body to be in fluid communication with the gas flow paths. The spools can be individually operated to move to respective open positions in the valve body in response to a pulse gas (typically air) signal to selectively active (and/or deactivate) at least one of a plurality of gas flow paths. The plurality of gas flow paths can be actuated by directing the pulse pilot gas to cause a respective spool to translate between open and closed positions to thereby open and close gas flow paths (block 110). Hyperpolarized gas is delivered to a subject during a NMR and/or MRI session or procedure via the gas flow path(s) defined by the spool positions in the valve body (block 120).

In certain embodiments, the pilot gas signal can have a pulse length between about 10-40ms, and typically between about 20-30ms (block 105).The pilot gas signal can be provided as a gas stream having a pressure between about 200-400 Pa [30-60 psi]. The gas delivery valve can be configured to operate with predetermined inhale, exhale, and breath-hold sequences **(block 112).** The gas delivery valve can be configured to ventilate hyperpolarized gas alone, hyperpolarized gas combined *in situ* with a selected non-polarized gas, and a non-polarized gas alone. The *in situ* combination can be caused by the positions of the spools in the valve body. The spools can be actuated by the steep rising edge of the pilot pressure curve associated with the pulsed pilot gas signal **(block 108).** The gas delivery valve may be adapted to accommodate or deliver at a breath rate of at least 150 BPM, typically up to at least up to about 180 BPM **(block 122).** In certain embodiments, the gas delivery valve may accommodate a breath rate of at least about 200 BPM (peak inspiration).

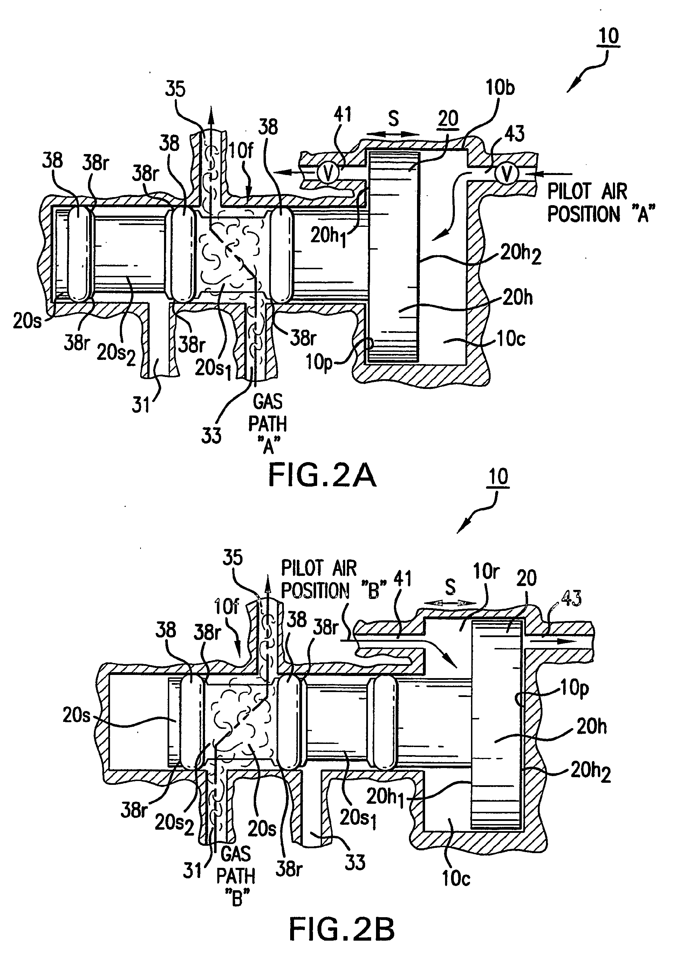

**Figures 2A** and **2B** illustrate one embodiment of the gas delivery valve **10.** The gas delivery valve **10** has a body **10b** with passages that define at least one gas flow path (the gas flow path or paths referred to generally as feature **10f)** and a receptacle **10r** for a spool **20.** Although shown as one spool and two different gas inlet ports (for Gas A and Gas B, respectively), multiple spools can be used in series and/or parallel to achieve different gas flow path options. In addition, the valve body and spool may be configured for an "on-off' operation of a single gas to deliver or hold back a single gas (typically hyperpolarized gas) and/or to open and close the single gas flow path (not shown) for controlled inhalation delivery to a subject. As will be discussed below, the gas delivery valve **10** can also be configured to select between inhale, exhale, and/or breath-hold ventilation operations.

Referring again to **Figure 2A****,** as shown, the spool **20** includes a head **20h** and a stem segment **20s.** The valve body receptacle **10r** includes pilot gas ports **41, 43** each positioned on opposing sides of the spool head portion **10c** of the receptacle **10r** so as to be facing opposing sides of the spool head **20h₁, 20h₂,** respectively. For ease of discussion, the term "pilot air" will be used instead of "pilot gas" in the description that follows. However, it is noted that although air is typically the pilot gas employed, other gases may be used to actuate the spool(s) as desired. Further, other fluids, including liquids, may also be used to actuate the spools in certain applications, although gas signals will typically provide faster transmissions.

In operation, pilot air is directed to enter either port **43** or **41** and apply pressure to the respective face of the spool head **20h.** Increasing and decreasing the pilot air pressure will adjust the speed (faster and slower, respectively). The pressure from the pilot air against the spool head **20h** forces the spool **20** to translate away from the pilot air input port. The translation positions the spool stem **20s** in different operative and/or inoperative locations in the valve body **10b.** In operation, the spool **20** translates (back and forth) to open and close selected gas flow paths in response to pilot gas transmitted into the valve body cavity of the spool head. The receptacle **10r** for the spool head **20h** is sized and configured so that the head of the spool **20h** can translate a desired stroke distance "S" to open and close one or more gas flow paths.

**Figure 2A** illustrates the pilot air being directed into port **43** on the outer face of the spool head **20h₂** to force the spool **20** to travel its stroke length "S" to position the inner face of the spool head **20h₁** against the receptacle perimeter **10p.** The stroke translation positions different portions of the stem **20s** in fluid communication with the gas exit port **35.** As shown, the stem **20s** includes two gas path segments, **20s₁** and **20s₂,** each sandwiched by seals **38.** The seals **38** are sized and configured to reside in recesses **38r** formed into the valve body at different locations along the spool travel path. The seals **38** may be O-rings or other suitable sealant members, formed of a material that inhibits depolarization that can enclose opposing portions of the stem segments **20s₁, 20s₂** during engagement of the selected gas flow path. Other sealing arrangements can also be used, such as by configuring the valve body and the stem to sealably matably engage at desired positions as will be understood by one of skill in the art. In **Figure 2A****,** the spool **20** is in a position in which segment **20s₁** is located so that gas path entry port **33** is in fluid communication with exit port **35** to open gas path "A" to deliver gas A through the gas delivery valve **10.** Either Gas path A or B is typically for supplying hyperpolarized gas (such as a hyperpolarized noble gas including, but not limited to, hyperpolarized ¹²⁹Xe or ³He).

**Figure 2B** illustrates pilot air being directed into port **41** with the outer face of the spool head **20h₂** being translated to reside against the perimeter **10p** of the valve body receptacle **10r.** This movement positions stem segment **20s₂** proximate exit port **35** to allow port **31** to be in fluid communication with the gas exit port **35** across the spool stem **20s** to open gas path B and close gas path A.

It is noted that, although shown in **Figures 2A** and **2B** as having only two gas flow inlet ports **(31,33)**, other numbers of valve body gas ports can be used (one or three, four or more). Further, the stem **20s** can be reconfigured to concurrently release gas A and gas B (combined), or to deliver other gases. Thus, additional valve ports can be added to allow porting in series or parallel. In addition, the spool head **20h** may be configured to have other stroke patterns such that the spool head **20h** surfaces are not required to stop against the perimeter **10p** of the valve body receptacle to actuate desired gas flow paths **10f** as will be understood by those of skill in the art.

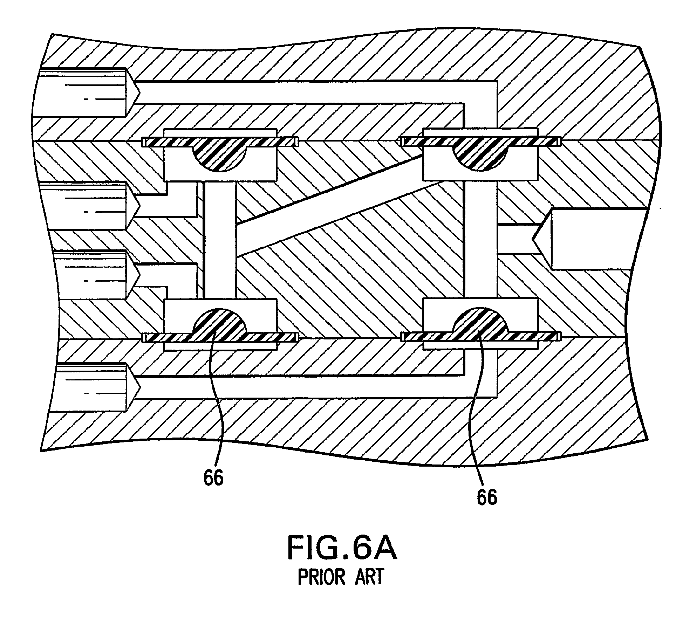

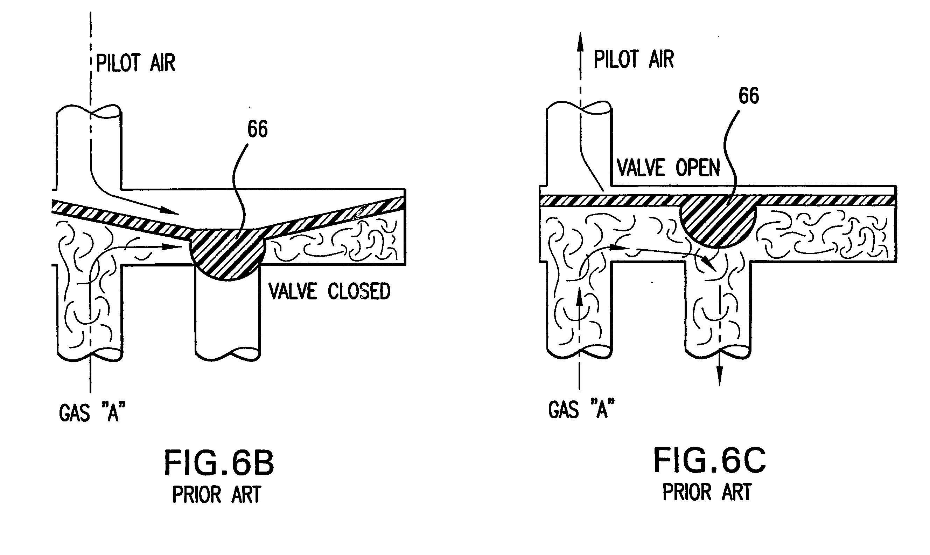

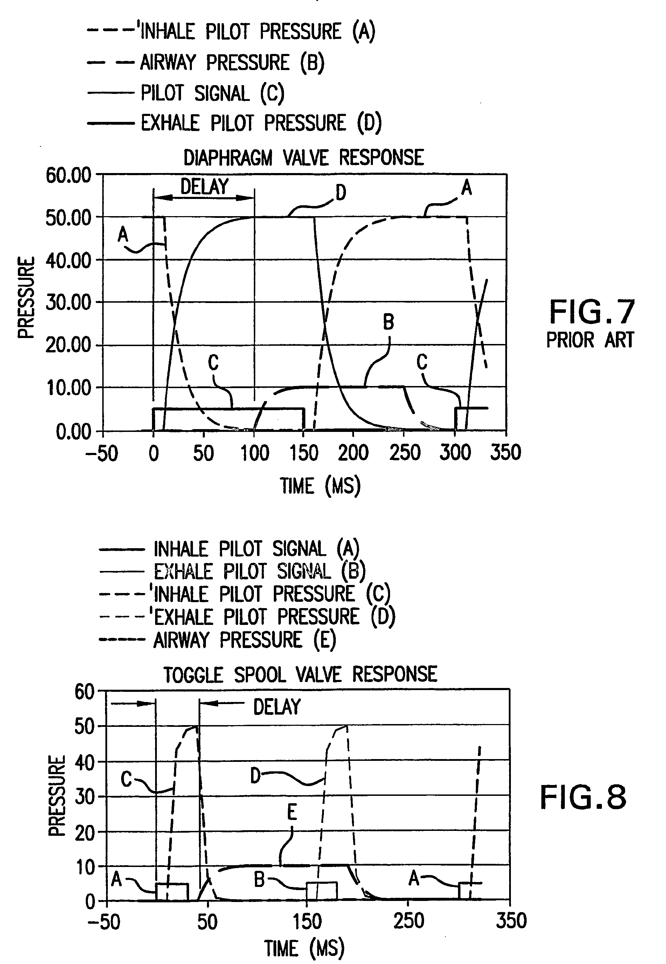

In certain embodiments, the spools are double acting spools that allow a single pulse of pilot air to actuate the valve to cause the spool **20** to move to the desired position and open or close the selected gas flow path. The pilot pressure can be released to atmosphere in between alternating pulses of pilot air to "toggle" the spool **20** from "inhale" to "exhale." The spool actuation (back and forth) either opens and/or closes one or more gas paths. The actuation can occur during the steep rinsing edge of the pilot pressure curve as shown, for example, in **Figure 8****.** The rising edge operation is in contrast to conventional diaphragm **66** valve configurations (*see, e.g.,* **Figures 6A-6C****)** that typically actuate the diaphragm to move to its operative position late in the falling edge portion of the response curve. An exemplary diaphragm pressure response curve is shown in **Figure 7****.** Thus, the spool configuration pressure at which the spool actuates and the gas path opens and/or closes occurs earlier in the pressure response cycle than with the diaphragm valve.

**Figure 8** illustrates that the length or pulse duration of the pilot signal is typically less than about 30ms, with the spool actuation delay being about 40ms or less. The response delay can be the same for either inhale or exhale pilot signals. As shown, the inhale and exhale pilot pressure can be substantially the same. The spool actuation response is relatively quick (less than about 50ms, and typically less than about 40ms) and acts with less than half the delay associated with conventional diaphragm valve configurations as shown in **Figure 7****.** The spool can be actuated with a response pressure that is between about 200-400 Pa [30-60 psi].

The volume of the pilot air circuit can be sized for quick response by optimizing the volume, port diameters and the like to provide a reduced spool stroke cycle. In certain embodiments, the spool stroke can be about 4mm [0.125 inches], the pilot circuit volume can be about 62 ml per cm [0.00306 in³per inch] of tubing. For example for 5 m [180 inches] of tubing line, the pilot circuit volume may be about 9 ml [0.5508 in³]. The pilot port diameters may be about 1,5 mm [0.0625 inches]. The spool head size may be about 15 mm [0.625 inches] (diameter). In certain embodiments, the spool head is sized to have an area of between about 0,3-3 cm² [0.05-0.45 in²]. The spool can be configured to weigh less than about 1 gram.

Referring back to **Figure 2A****,** the spool is operably associated with two pilot valves "V" (one in fluid communication with pilot-port **41** and the other in fluid communication with pilot port **43**). The two pilot valves can be electric, three-way, normally closed solenoid valves. After the command pilot pulse signal is transmitted to the desired spool (or spools), typically with a pulse of between about 20-30ms, but generally under about 40ms, the normally closed pilot valve sending the command pilot pulse can be vented to atmosphere so that the command pilot pressure does not offer resistance to the opposing pilot air when it is actuated to drive the spool back to its former position. The dual acting spool configuration can be operated so that the pilot pressure is always on to provide a biasing mechanism to cause the spool **20** to return to a neutral or normal operating position after the pilot pulse signal is transmitted. The air is always directed to one side of the other to bias the location of the spool without requiring the use of a spring member. In other embodiments, a polarization friendly spring (formed of a resilient non-ferromagnetic metal or elastomeric material) can be used to help bias the spool to return to a desired position after transmission of the pilot command signal (not shown).

The valve body and its internal components can be formed from and/or coated with a material or materials selected for the ability to inhibit depolarization of hyperpolarized gas (such as due to relaxation attributed to gas interaction therewith). Coatings such as sol-gel coatings, deuterated polymer coatings, metal film coatings and other coatings and non-magnetic materials that inhibit depolarization have also been proposed. *See, e.g.,* U.S. Patent Application No. 09/485,476 and U.S. Patent Nos. 5,612,103 and 6,423,387.

For example, the valve body and/or components may be formed of materials such as, but not limited to, aluminum, TEDLAR, TEFLON, PTFE, DELRIN (acetal), and the like. Care should be taken to reduce the sources of friction by providing a smooth surface finish, and reducing the number of O-rings or selecting the O-ring material to reduce friction. In addition, the valve body can be fabricated to tolerances to provide sufficient seals and yet provide reduced friction between the spool and valve body receptacle. Thus, the valve body bore finishes, O-ring compression, and lubricants may all be considered to reduce the sources of friction.

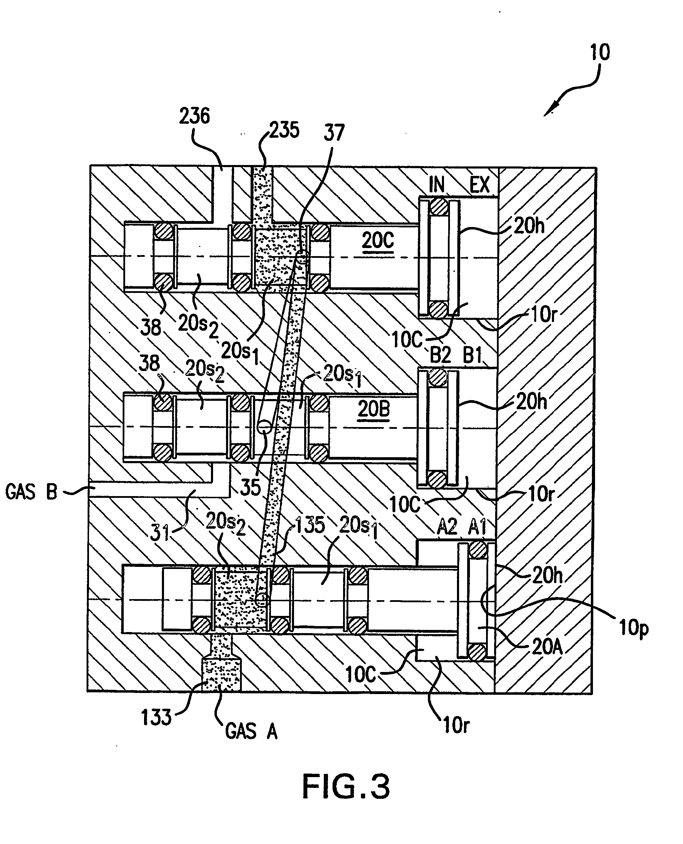

**Figure 3** illustrates a valve **10** having a valve body **10b** that defines three spool receptacles **10r** each for accommodating one of spools **20A, 20B, 20C,** respectively. Control of the input of Gas A into the valve **10** is controlled by a first spool **20A** that is positioned to control the gas port **133** that is in fluid communication with the external Gas A source. Valve body **10b** defines a gas flowpath **135** extending in fluid communication between the spool receptacles for spools **20A** and **20C** while extending about the spool receptacle for spool **20B. Spool 20A** has two operative positions, **A1** and **A2.** In the **A1** position shown (with the spool head **20h** residing against perimeter **10p** at the outermost portion of the spool cavity **10c),** Gas A is allowed to travel from the spool receptacle for spool 20A, through flowpath **135** into the spool receptacle for spool **20C,** and out the exit port **235.** Valve body **10b** defines exit port **235** for conducting gas to a subject for inhaling and from a subject on exhaling. Suitable gas conduits, not shown, are contemplated to carry the gases to and from the appropriate ports of valve **10.**

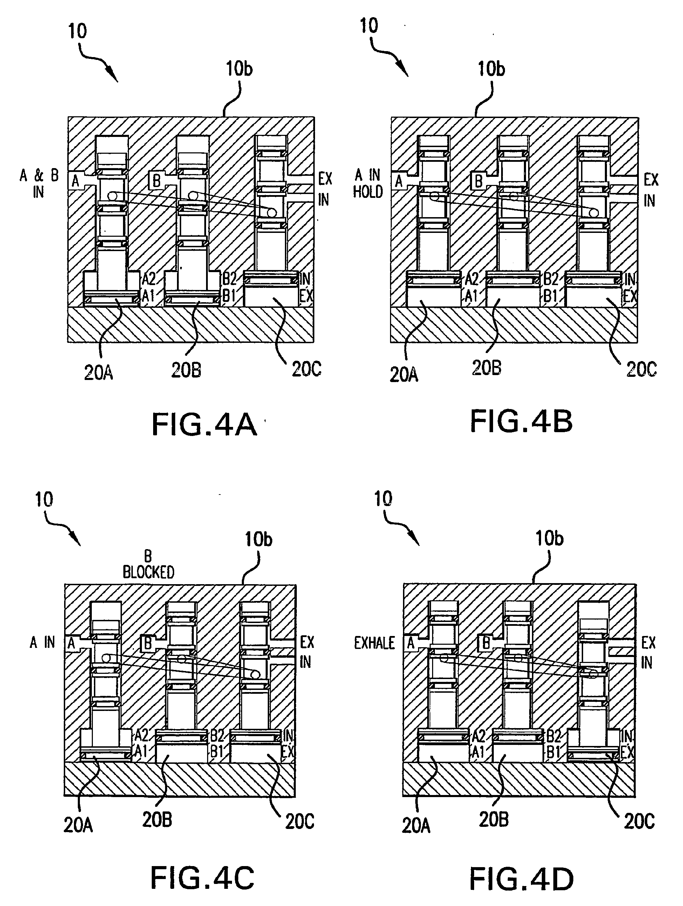

Similarly, the flow of Gas B from an external source and through the Gas B input port **31** to exit port **235** is controlled by the positioning of second spool **20B.** The second spool **20B** also has two operative positions, **B1** and **B2.** Valve body **10b** defines a second elongate flowpath **35** extending in fluid communication between the spool receptacle for spool **20B** and an aperture **37** opening into the spool receptacle for spool **20C.** Aperture **37** desirably is located opposite the open end of flowpath **135** from the spool receptacle for spool **20A,** although they may be arranged differently, or even co-extensively. The positioning of the spool segments **20s₁, 20s₂** of spools **20A** and **20B** can be adjusted to allow Gas B to be output alone or combined with Gas A, as will be further described for Figures 4A-4H hereinbelow. The second **spool 20B** is shown in position **B2** with the Gas B input port **31** isolated from flowpath **35** and Gas A allowed to pass all the way through to exit port **235.** When spool **20B** is in the **B1** position, Gas B will be able to travel through flowpath **35** and out exit port 235.

The third spool **20C** is configured to control the inhale and exhale configuration of the valve **10.** As such, the third **spool 20C** has an IN position and an EX position. The valve body **10b** defines an exit ports **235** and **236** in fluid communication with the spool receptacle 10r for spool 20C. The position of the third spool **20C** cooperates with the associated valve receptacle **10r** to either direct flow from about spool segment **20s₂** and out exit port 235 when spool 20C is in the IN (or inhale) position, or from exit port 235, about spool segment **20s₁** and out exit port **236** when spool 20C is in the EX (or exhale) position, as desired. The gas flow path depicted in **Figure 3** is illustrated by the dark shading extending from Gas A input port 133 to the valve **10** exit inhale port **135** that is actuated to expel Gas A (alone) through the ventilation inhale port **135.**

Whereas flowpaths **35** and **135,** which are considered to extend outside the plane of the view depicted in Figure 3 are depicted in Figure 3, the preset invention contemplates that valve body 10b further defines pilot air ports (not shown) for directing air against either side of the spool heads for spools **20A, 20B,** and **20C** which are also located outside the plane of the view. These pilot air ports would be configured to incorporate the features of ports **41** and **43** described in Figures 2A and 2B. A pair of opposing pilot air ports desirably provide pilot air to the opposing faces of each spool head **20h.** The valving for such ports are desirably located outside of valve body **10b,** although such valves may be incorporated into valve body **10b** as well. The present invention further contemplates that each spool may be acted upon by a single pilot air port which directs air against one face of each spool head **20h** to move the spool in a first direction to its first position and draws air out of bore **10C** for each spool to force the spool to travel the opposite direction to its second position. Alternatively, opposing pilot air ports may act in unison to actively force air into, or out of, bore **10C** in order to urge the spool to the desired position.

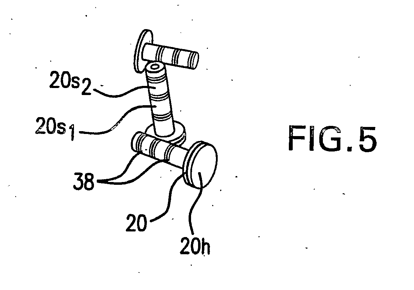

**Figure 5** depicts the three spools 20 of Figure 3 specifically and of the present invention generally. **Figure 5** further illustrate spool segments **20s₁, 20s₂** of each spool 20, as well as spool head **20h** and the gaskets **38** which sealingly engage the valve body 10b for directing flow therethrough.

Figures **4A-4H** illustrate examples of different valve positions of a three-spool configuration that can be used to provide selection of ventilation breath outputs and/or inputs according to embodiments of the present invention. For discussion purposes, Gas A will be described as a hyperpolarized gas and Gas B as a non-polarized gas. In operation, a reverse configuration can be used. The term "breath-hold" means that the gas or gases are held in the lungs for a breath-hold time to facilitate bio-uptake of the gas an/or allow a stronger hyperpolarized gas signal other normal respiration. The breath-hold duration may be between 5-30 seconds.

As shown and further described below, the valve **10** can be actuated to accommodate or provide (a) Gas "A" inhale; (b) Gas "B" inhale; (c) Gas "A" + Gas "B" inhale; (d) exhale; (e) partial exhale and breath hold; (f) Gas "A" inhale and breath hold; (g) Gas "B" inhale and hold; and (h) Gas "A" + Gas "B" inhale and breath hold. The valve spool configuration can be adjusted to provide all or selected ones of these ventilation breath inputs/outputs and may use lesser or greater numbers of spools and/or respective spool segments as well as different porting configurations to provide the desired breath outputs/inputs.

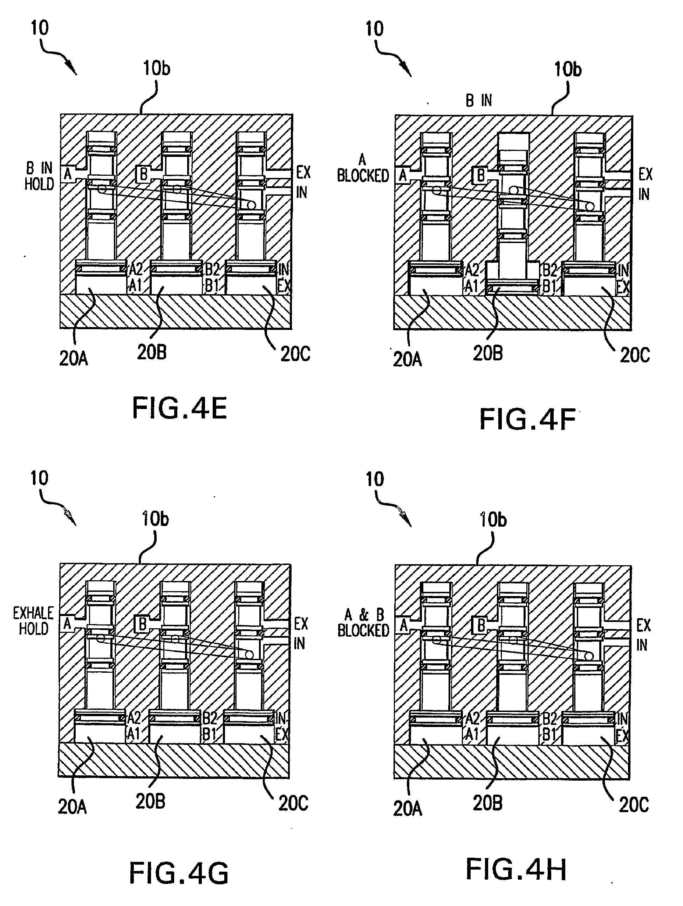

**Figure 4A** illustrates the operative position of the three spools to provide Gas "A" + Gas "B" inhale. **Figure 4B** illustrates Gas "A" output in an inhale breath-hold position. **Figure 4C** shows the positions of the spools for Gas "A" inhale. **Figure 4D** shows the spool positions for exhale; **Figure 4E** shows the spools in position for Gas "B" inhale and hold. **Figure 4F** shows the positions of the spools for Gas "A" blocked and **Figure 4G** shows an exhale hold configuration. **Figure 4H** illustrates a Gas "A"+ Gas "B" hold configuration.

The valve spool positions and sequencing of operation can be automated and controlled by a computer control program. The actuation or piloting of the valve spools **20A, 20B, 20C,** can be driven through the computer control program by six (6) electric three-way normally closed solenoid valves. Examples of suitable solenoid valves include, Clippard EC-#M-12V, Cincinnati, OH, and MAC 42A-AMP-000-GDDB-1BE, Wixom, MI. In certain embodiments, a command is given to actuate an individual spool to a desired position. The command triggers the transmission of a 20-30ms pilot command pulse signal to the individual spool. The single command pulse toggles the spool to the desired position. After the pulse, the normally closed solenoid valve vents to atmosphere so that the pilot pressure for that spool does not generate undue resistance to the opposing pilot gas (air) when it is actuated to drive the spool back to its previous position.

The hyperpolarized gas may be a hyperpolarized noble gas such as ³He and/or ¹²⁹Xe. The non-polarized gas may be a mixture of non-polarized gases. The non-polarized gas can be selected to inhibit depolarization of the hyperpolarized gas, and may be also selected for biocompatibility. Examples of suitable non-polarized gases include, but are not limited to, nitrogen, xenon, and helium.

The valve body **10b (****Figure 4****)** can be configured to hold a plurality of individually operable spools in close proximity. The valve spools or translating components may be configured so that the centerlines of adjacent receptacles are less 10 components may be configured so that the centerlines of adjacent receptacles are less than 25 mm [1inch] apart, and, in certain embodiments, are about 12 mm [0.5 inches] apart. During operation, the valve can be configured to reduce the number of breath cycles that are required for switching between gases output to a subject. In addition, the spool configuration is such that dead volume for the inhaled gas(es) can be reduced. Dead volume is the volume within the valve that needs to be filled with gas before the gas will reach the subject. The smaller the volume, the less gas is "wasted" inside the valve. Reducing the dead volume is particularly desirable when dispensing hyperpolarized gas because the hyperpolarized gas fills the volume of the flow path from source to subject and any polarized gas remaining in the dead volume may become unpolarized or decay to an undesirable polarization level, and must be displaced before suitably polarized gas can reach the subject on the next polarized breath. In certain embodiments, the dead volume for the gas delivery valve can be about 0.445 ml. In particular embodiments, the dead volume may be even less, such as on the order of about 0.223 ml.

The foregoing is illustrative of the present invention and is not to be construed as limiting thereof Although a few exemplary embodiments of this invention have been described, those skilled in the art will readily appreciate that many modifications are possible in the exemplary embodiments without materially departing from the novel teachings and advantages of this invention. Accordingly, all such modifications are limited by the scope of this invention as defined in the claims. In the claims, means-plus-function clauses, where used, are intended to cover the structures described herein as performing the recited function and not only structural equivalents but also equivalent structures. Therefore, it is to be understood that the foregoing is illustrative of the present invention and is not to be construed as limited to the specific embodiments disclosed, and that modifications to the disclosed embodiments, as well as other embodiments, as long as they are included within the scope of the appended claims.

## Claims

1. A gas delivery valve [10] for a ventilator configured to supply hyperpolarized gas to a subject, the gas delivery valve [10] comprising:
a valve body [10b] having at least one gas flow path [10f] extending between at least one gas inlet port [31, 33] and at least one gas exit port [35] and at least one spool receptacle [10r], one of the at least one gas inlet ports [31,33] being in fluid communication with a hyperpolarized gas source;
at least one spool member [20] disposed in a respective one of said at least one spool receptacles [10r] of said valve body [10b], said spool member [20] configured and sized to cooperate with said valve body [10b] to selectively open and close the at least one gas flow path [10f]; and
at least one pilot fluid port in fluid communication with said spool receptacle [10r],
wherein, in operation, a pilot fluid command pulse signal having an associated pressure and duration is transmitted into said pilot fluid port thereby forcing said spool [20] to translate an actuation distance in a predetermined direction in said valve body receptacle [10r] to open and/or close the at least one gas flow path [10f].

2. A gas delivery valve [10] according to Claim 1, wherein the valve body [10b] has a plurality of controllably selectable different gas flow paths [10f] therein, each having an associated gas inlet port [31, 33], one of said gas inlet ports [31, 33] being in fluid communication with the hyperpolarized gas source and a different one of said gas inlet ports [31,33] being in fluid communication with a non-polarized gas source, wherein the spool member [20] comprises a plurality of spaced a part stem segments [20s] with a seal member [38] disposed therebetween, and Wherein the position of the spool [20] in the valve body [10b] aligns a respective one of the stem segments [20s] with a respective one of the gas inlet ports [31, 33] and the gas exit port [35] to open a respective gas flow path [10f] to deliver the hyperpolarized gas or the non-polarized gas provided from the aligned gas inlet port [31, 33] to the gas exit port [35] to a subject.

3. A gas delivery valve [10] according to Claim 1, wherein the spool [20] comprises a spool head [20h] and an elongated stem [20s].

4. A gas delivery valve [10] according to Claim 3, wherein the spool receptacles [10r] in the valve body [10b] includes a spool head portion [10c] that has an axial length that is longer than the spool head [20h], and wherein the length of the receptacle spool head portion [10c] defines the stroke actuation translation distance [S] of the spool [20].

5. A gas delivery valve [10] according to Claim 4, wherein the at least one pilot fluid port is two fluid pilot ports [41, 43], each positioned on opposing sides of the spool receptacle portion [10c] so as to be proximate a respective one of the major surfaces [20h₁, 20h₂] of the spool head [20h] during operation.

6. A gas delivery valve [10] according to Claim 5, wherein the pilot fluid ports [41,43] are pilot gas ports, said valve further comprising a pilot gas source configured to provide a pilot command pulse signal having a duration that is less than about 40ms.

7. A gas delivery valve [10] according to Claim 6, wherein the pilot command pulse signal generates a spool pressure response profile that has a delay measured from the start of the pilot command pulse signal to the maxima of the pressure response profile that is less than about 50ms.

8. A gas delivery valve [10] according to Claim 7, wherein the spool pressure response profile has a rising initial portion that rises to between about 200-400 Pa [30-60 psi.].

9. A gas delivery valve [10] according to Claim 8, wherein, in operation the spool [20] travels the stroke actuation distance [S] in the valve body [10b] in response to the rising initial portion of the spool pressure response profile.

10. A gas delivery valve [10] according to Claim 1, wherein at least one gas flow path [10f] of the valve body [10b] is a plurality of different gas flow paths [10f], wherein the valve body [10b] comprises a plurality of spaced apart spool receptacles [10r] and a corresponding number of spools [20], each spool receptacle [10r] comprising two pilot ports in fluid communication with a pilot gas source, and wherein respective spools [20] are configured to selectively open and close gas flow paths [10f] in the valve body [10b] in response to pilot command pulse signals transmitted thereto so as to controllably serially or concurrently output a plurality of different gases from the gas delivery valve [10].

11. A gas delivery valve [10] according to Claim 10, wherein the valve body comprises an exhale port proximate the exit port [35], wherein the plurality of receptacles [10r] and spools [20] includes, a first receptacle [10r] and a corresponding first spool [20A] disposed proximate the exhale port and the exit port [35], a second receptacle [10r] and second spool [20B] spaced apart from and in fluid communication with the first spool [20A], the second receptacle [10r] having gas inlet port B [31] to supply non-polarized Gas B during operation, and a third receptacle [10r] and third spool [20C] spaced apart from the first [20A] and second [20B] spools and in fluid communication with the second spool [20B], the third receptacle [10r] having gas inlet port A [33] to supply hyperpolarized Gas A during operation, and wherein the gas inlet port to the first receptacle [10r] is either or both the exit ports [35, 235] from the second and third receptacles [10r]

12. A gas delivery valve [10] according to Claim 10, wherein the gas flow paths [10F] comprise a first inhale gas flow path, a second exhale gas flow path, and a third breath-hold gas flow path, each of which are remotely selectable gas flow paths.

13. A gas delivery valve [10] according to Claim 10, wherein the spools [20] and valve body [10b] are arranged to allow hyperpolarized gas, non-polarized gas, or a combination of the hyperpolarized gas and non-polarized gas, to be output from the inhale and/or breath-hold gas flow paths [10f].

14. A gas delivery valve [10] according to Claim 6, wherein the spool [20] reciprocates between first and second operative positions [A1, A2], said valve further comprising two normally closed solenoid valves [V], one operably associated with each of the pilot fluid ports [41, 43] and the pilot gas source, the solenoid valves configured to control the actuation of the spool [20] between the first and second positions, wherein, in operation, after a command pilot pulse signal is transmitted to a spool head via a selected one of the pilot ports [41, 43] to force the spool [20] to translate to the second position [A2] from the first position [A1], the respective normally closed pilot valve [V] associated with the selected pilot port vents to atmosphere so that pilot pressure in the spool head receptacle portion [10c] is reduced to inhibit interference with the opposing pilot gas signal from the other pilot port when the valve spool is actuated to drive the spool [20] back to its first position [A1].

15. A gas delivery valve [10] according to Claim 14, further comprising a computer module with computer program code that remotely controls the sequence of pilot command signals to respective spools [20] to actuate the spools [20] and automatically open and close desired gas flow paths [10f] in the valve body [10b] to ventilate with the desired sequence of hyperpolarized gas and non-polarized gas.

16. A gas delivery valve [10] according to Claim 11, further comprising a plurality of conduits outwardly extending from the valve body [10b] to define a plurality of gas flow paths [10f], the plurality of conduits including two pilot gas flow path conduits for each spool head receptacle portion [10c] in the valve body [10b], at least one hyperpolarized gas conduit extending from the hyperpolarized gas source to a corresponding gas inlet port [33] in the valve body [10b], and at least one non-polarized gas conduit extending from a non-polarized gas source to a corresponding gas inlet port [31] in the valve body [10b].

## Patentansprüche

1. Gaszuführungsventil [10] für einen Ventilator, gestaltet, um einem Subjekt hyperpolarisiertes Gas zuzuführen, wobei das Gaszuführungsventil [10] umfasst:
einen Ventilkörper [10b] mit mindestens einem Gasströmungsweg [10f], der sich zwischen mindestens einem Gaseinlassanschluss [31, 33] und
mindestens einem Gasauslassanschluss [35] erstreckt, und mit mindestens einem Schieberaufnahmeteil [10r], wobei einer des mindestens einen Gaseinlassanschlusses [31, 33] in fluider kommunikativer Verbindung mit einer Quelle hyperpolarisierten Gases ist;
mindestens ein Schieberelement [20], das in einem jeweiligen des mindestens einen Schieberaufnahmeteils [10r] des Ventilkörpers [10b] angeordnet ist,
wobei das Schieberelement [20] so gestaltet und bemessen ist, dass es mit dem Ventilkörper [10b] zusammenwirkt, um den mindestens einen Gasströmungsweg [10f] selektiv zu öffnen und zu schließen; und
mindestens einen Pilotfluidanschluss in fluider kommunikativer Verbindung mit dem Schieberaufnahmeteil [10r],
wobei, bei Betrieb, ein Pilotfluidbefehlsimpulssignal mit zugehörigem Druck und zugehöriger Dauer, in den Pilotfluidanschluss übertragen wird, um dadurch den Schieber [20] zu zwingen, eine Betätigungsstrecke in einer vorbestimmten Richtung im Ventilkörperaufnahmeteil [10r] geradlinig zurückzulegen, um den mindestens einen Gasströmungsweg [10f] zu öffnen und/oder zu schließen.

2. Gaszuführungsventil [10] nach Anspruch 1, wobei der Ventilkörper [10b] eine Mehrzahl verschiedener Gasströmungswege [10f], die steuerbar auswählbar sind, in demselben aufweist, wobei jeder einen zugehörigen Gaseinlassanschluss [31, 33] aufweist, einer der Gaseinlassanschlüsse [31, 33] in fluider kommunikativer Verbindung mit der Quelle hyperpolarisierten Gases ist und ein anderer der Gaseinlassanschlüsse [31, 33] in fluider kommunikativer Verbindung mit einer Quelle nicht polarisierten Gases ist, wobei das Schieberelement [20] eine Mehrzahl voneinander beabstandeter Schaftsegmente [20s] mit einem zwischen denselben angeordneten Dichtungselement [38] umfasst, und wobei die Position des Schiebers [20] im Ventilkörper [10b] ein jeweiliges der Schaftsegmente [20s] an einem jeweiligen der Gaseinlassanschlüsse [31, 33] und dem Gasauslassanschluss [35] ausrichtet, um einen jeweiligen Gasströmungsweg [10f] zu öffnen, um das hyperpolarisierte Gas oder das nicht polarisierte Gas, das aus dem ausgerichteten Gaseinlassanschluss [31, 33] zum Gasauslassanschluss [35] geliefert wird, einem Subjekt zuzuführen.

3. Gaszuführungsventil [10] nach Anspruch 1, wobei der Schieber [20] einen Schieberkopf [20h] und einen länglichen Schaft [20s] umfasst.

4. Gaszuführungsventil [10] nach Anspruch 3, wobei das Schieberaufnahmeteil [10r] im Ventilkörper [10b] einen Schieberkopfabschnitt [10c] beinhaltet, der eine axiale Länge aufweist, die größer ist als der Schieberkopf [20h], und wobei die Länge des Aufnahmeteilschieberkopfabschnitts [10c] die Hubbetätigungstranslationsstrecke [S] des Schiebers [20] festlegt.

5. Gaszuführungsventil [10] nach Anspruch 4, wobei der mindestens eine Pilotfluidanschluss in zwei Fluidpilotanschlüssen [41, 43] besteht, die je auf gegenüberliegenden Seiten des Schieberaufnahmeteilabschnitts [10c] positioniert sind, um nächst einer jeweiligen der Hauptoberflächen [20h₁, 20h₂] des Schieberkopfs [20h] während des Betriebs zu sein.

6. Gaszuführungsventil [10] nach Anspruch 5, wobei die Pilotfluidanschlüsse [41, 43] Pilotgasanschlüsse sind, wobei das Ventil weiterhin eine Pilotgasquelle umfasst, die gestaltet ist, um ein Pilotbefehlsimpulssignal mit einer Dauer zu liefern, die kürzer ist als etwa 40 ms.

7. Gaszuführungsventil [10] nach Anspruch 6, wobei das Pilotbefehlsimpulssignal ein Schieberdruckreaktionsprofil erzeugt, das eine vom Beginn des Pilotbefehlsimpulssignals zu den Maxima des Druckreaktionsprofils gemessene Verzögerung aufweist, die kürzer ist als etwa 50 ms.

8. Gaszuführungsventil [10] nach Anspruch 7, wobei das Schieberdruckreaktionsprofil einen steigenden Anfangsabschnitt aufweist, der auf zwischen etwa 200 - 400 Pa ansteigt.

9. Gaszuführungsventil [10] nach Anspruch 8, wobei, bei Betrieb, der Schieber [20] die Hubbetätigungsstrecke [S] im Ventilkörper [10b] in Reaktion auf den steigenden Anfangsabschnitt des Schieberdruckreaktionsprofils zurücklegt.

10. Gaszuführungsventil [10] nach Anspruch 1, wobei mindestens ein Gasströmungsweg [10f] des Ventilkörpers [10b] in einer Mehrzahl verschiedener Gasströmungswege [10f] besteht, wobei der Ventilkörper [10b] eine Mehrzahl voneinander beabstandeter Schieberaufnahmeteile [10r] und eine entsprechende Anzahl von Schiebern [20] umfasst, wobei jedes Schieberaufnahmeteil [10r] zwei Pilotanschlüsse in fluider kommunikativer Verbindung mit einer Pilotgasquelle umfasst, und wobei jeweilige Schieber [20] gestaltet sind, um Gasströmungswege [10f] im Ventilkörper [10b] in Reaktion auf dahin übertragene Pilotbefehlsimpulssignale selektiv zu öffnen und zu schließen, um eine Mehrzahl verschiedener Gase aus dem Gaszuführungsventil [10] regelbar seriell oder gleichzeitig herauszulassen.

11. Gaszuführungsventil [10] nach Anspruch 10, wobei der Ventilkörper einen Ausatmungs-Anschluss nächst dem Auslassanschluss [35] umfasst, wobei die Mehrzahl von Aufnahmeteilen [10r] und Schiebern [20] ein erstes Aufnahmeteil [10r] und einen entsprechenden ersten Schieber [20A] beinhaltet, angeordnet nächst dem Ausatmungs-Anschluss und dem Auslassanschluss [35], ein zweites Aufnahmeteil [10r] und einen zweiten Schieber [20B], beabstandet von und in fluider kommunikativer Verbindung mit dem ersten Schieber [20A], wobei das zweite Aufnahmeteil [10r] einen Gaseinlassanschluss B [31] aufweist, um nicht polarisiertes Gas B während des Betriebs zuzuführen, und ein drittes Aufnahmeteil [10r] und einen dritten Schieber [20C], beabstandet vom ersten [20A] und zweiten [20B] Schieber und in fluider kommunikativer Verbindung mit dem zweiten Schieber [20B], wobei das dritte Aufnahmeteil [10r] einen Gaseinlassanschluss A [33] aufweist, um hyperpolarisiertes Gas A während des Betriebs zuzuführen, und wobei der Gasseinlassanschluss zum ersten Aufnahmeteil [10r] in entweder einem oder beiden der Auslassanschlüsse [35, 235] aus dem zweiten und dritten Aufnahmeteil [10r] besteht.

12. Gaszuführungsventil [10] nach Anspruch 10, wobei die Gasströmungswege [10f] einen ersten Einatmungs-Gasströmungsweg, einen zweiten Ausatmungs-Gasströmungsweg und einen dritten Atemanhaltungs-Gasströmungsweg umfassen, von denen jeder ein aus der Ferne wählbarer Gasströmungsweg ist.

13. Gaszuführungsventil [10] nach Anspruch 10, wobei die Schieber [20] und der Ventilkörper [10b] angeordnet sind, um zu ermöglichen, dass hyperpolarisiertes Gas, nicht polarisiertes Gas, oder eine Kombination aus dem hyperpolarisierten Gas und dem nicht polarisierten Gas, aus dem Einatmungs- und/oder Atemanhaltungs-Gasströmungsweg [10f] herausgelassen werden.

14. Gaszuführungsventil [10] nach Anspruch 6, wobei sich der Schieber [20] zwischen einer ersten und zweiten Betriebsposition [A1, A2] hin- und herbewegt, wobei das Ventil weiterhin zwei normal geschlossene Magnetventile [V] umfasst, von denen eines operativ zu jedem der Pilotfluidanschlüsse [41, 43] und der Pilotgasquelle gehört, wobei die Magnetventile gestaltet sind, um die Betätigung des Schiebers [20] zwischen der ersten und zweiten Position zu steuern, wobei, bei Betrieb, nachdem ein Befehlspilotimpulssignal zu einem Schieberkopf über einen ausgewählten der Pilotanschlüsse [41, 43] übertragen worden ist, um den Schieber [20] zu zwingen, sich geradlinig aus der ersten Position [A1] zur zweiten Position [A2] zu bewegen, das jeweilige normal geschlossene Pilotventil [V], das zu dem ausgewählten Pilotanschluss gehört, zur Atmosphäre entlüftet, so dass Pilotdruck im Schieberkopfaufnahmeteilabschnitt [10c] verringert wird, um eine Interferenz mit dem entgegengesetzten Pilotgassignal vom anderen Pilotanschluss zu verhindern, wenn der Ventilschieber betätigt wird, um den Schieber [20] zurück zu seiner ersten Position [A1] zu treiben.

15. Gaszuführungsventil [10] nach Anspruch 14, weiterhin umfassend ein Computermodul mit Computerprogrammcode, der aus der Ferne die Sequenz von Pilotbefehlssignalen zu jeweiligen Schiebern [20] steuert, um die Schieber [20] zu betätigen und gewünschte Gasströmungswege [10f] im Ventilkörper [10b] automatisch zu öffnen und zu schließen, so dass mit der gewünschten Sequenz von hyperpolarisiertem Gas und nicht polarisiertem Gas zu ventiliert wird.

16. Gaszuführungsventil [10] nach Anspruch 11, weiterhin umfassend eine Mehrzahl von Leitungen, die sich vom Ventilkörper [10b] auswärts erstrecken, um eine Mehrzahl von Gasströmungswegen [10f] festzulegen, wobei die Mehrzahl von Leitungen zwei Pilotgasströmungswegleitungen für jeden Schieberkopfaufnahmeteilabschnitt [10c] im Ventilkörper [10b], mindest eine Leitung für hyperpolarisiertes Gas, die sich von der Quelle hyperpolarisierten Gases zu einem entsprechenden Gaseinlassanschluss [33] im Ventilkörper [10b] erstreckt, und mindestens eine Leitung für nicht polarisiertes Gas beinhaltet, die sich von einer Quelle nicht polarisieren Gases zu einem entsprechenden Gaseinlassanschluss [31] im Ventilkörper [10b] erstreckt.

## Revendications

1. Vanne d'administration de gaz (10) pour un appareil de ventilation configuré afin de délivrer du gaz hyperpolarisé à un sujet, la vanne d'administration de gaz (10) comprenant :
un corps de vanne (10b) comportant au moins un trajet d'écoulement de gaz (10f) s'étendant entre au moins un orifice d'entrée de gaz (31, 33) et au moins un orifice de sortie de gaz (35) et au moins un réceptacle de tiroir (10r), l'un des au moins un orifice d'entrée de gaz (31, 33) étant en communication fluidique avec une source de gaz hyperpolarisé ;
au moins un élément formant tiroir (20) disposé dans l'un respectif desdits au moins un réceptacle de tiroir (10r) dudit corps de vanne (10b), ledit élément formant tiroir (20) étant configuré et dimensionné afin de coopérer avec ledit corps de vanne (10b) de manière à ouvrir et fermer sélectivement le au moins un trajet d'écoulement de gaz (10f) ; et
au moins un orifice de fluide pilote en communication fluidique avec ledit réceptacle de tiroir (10r),
dans laquelle, en fonctionnement, un signal impulsionnel de commande de fluide pilote présentant une pression et une durée associées est transmis dans ledit orifice de fluide pilote forçant ainsi ledit tiroir (20) à se déplacer sur une distance d'activation dans une direction prédéterminée dans ledit réceptacle de corps de vanne (10r) afin d'ouvrir et/ou de fermer le au moins un trajet d'écoulement de gaz (10f).

2. Vanne d'administration de gaz (10) selon la revendication 1, dans laquelle le corps de vanne (10b) présente une pluralité de trajets d'écoulement de gaz différents (10f) pouvant être sélectionnés par commande à l'intérieur, chacun présentant un orifice d'entrée de gaz associé (31, 33), l'un desdits orifices d'entrée de gaz (31, 33) étant en communication fluidique avec la source de gaz hyperpolarisé et l'un différent desdits orifices d'entrée de gaz (31, 33) étant en communication fluidique avec une source de gaz non polarisé, dans laquelle l'élément formant tiroir (20) comprend une pluralité de segments formant manchon espacés (20s) avec un élément d'étanchéité (38) disposé entre eux, et dans laquelle la position du tiroir (20) dans le corps de vanne (10b) aligne l'un respectif des segments formant manchon (20s) avec l'un respectif des orifices d'entrée de gaz (31, 33) et l'orifice de sortie de gaz (35) afin d'ouvrir un trajet d'écoulement de gaz respectif (10f) de manière à délivrer le gaz hyperpolarisé ou le gaz non polarisé fourni à partir de l'orifice d'entrée de gaz aligné (31, 33) vers l'orifice de sortie de gaz (35) à un sujet.

3. Vanne d'administration de gaz (10) selon la revendication 1, dans laquelle le tiroir (20) comprend une tête de tiroir (20h) et un manchon allongé (20s).

4. Vanne d'administration de gaz (10) selon la revendication 3, dans laquelle le réceptacle de tiroir (10r) dans le corps de vanne (10b) comporte une partie de tête de tiroir (10c) qui présente une longueur axiale qui est supérieure à celle de la tête de tiroir (20h), et dans laquelle la longueur de la partie de tête de tiroir de réceptacle (10c) définit la distance de déplacement d'activation de course (S) du tiroir (20).

5. Vanne d'administration de gaz (10) selon la revendication 4, dans laquelle le au moins un orifice de fluide pilote est constitué par deux orifices de fluide pilote (41, 43), chacun étant positionné sur les côtés opposés de la partie de réceptacle de tiroir (10c) de manière à être à proximité de l'une respective des surfaces principales (20h₁, 20h₂) de la tête de tiroir (20h) au cours du fonctionnement.

6. Vanne d'administration de gaz (10) selon la revendication 5, dans laquelle les orifices de fluide pilote (41, 43) sont des orifices de gaz pilote, ladite vanne comprenant, en outre, une source de gaz pilote configurée afin de délivrer un signal impulsionnel de commande pilote présentant une durée qui est inférieure à 40 ms.

7. Vanne d'administration de gaz (10) selon la revendication 6, dans laquelle le signal impulsionnel de commande pilote produit un profil de réponse de pression de tiroir qui présente un délai mesuré à partir du début du signal impulsionnel de commande pilote jusqu'au maximum du profil de réponse de pression qui est inférieur à 50 ms.

8. Vanne d'administration de gaz (10) selon la revendication 7, dans laquelle le profil de réponse de pression de tiroir présente une partie initiale croissante qui s'élève entre 200 et 400 Pa (30 et 60 psi) environ.

9. Vanne d'administration de gaz (10) selon la revendication 8, dans laquelle, en fonctionnement, le tiroir (20) se déplace sur la distance d'activation de course (S) dans le corps de vanne (10b) en réponse à la partie initiale croissante du profil de réponse de pression de tiroir.

10. Vanne d'administration de gaz (10) selon la revendication 1, dans laquelle au moins un trajet d'écoulement de gaz (10f) du corps de vanne (10b) est une pluralité de différents trajets d'écoulement de gaz (10f), dans laquelle le corps de vanne (10b) comprend une pluralité de réceptacles de tiroir espacés (10r) et un nombre correspondant de tiroirs (20), chaque réceptacle de tiroir (10r) comprenant deux orifices pilotes en communication fluidique avec une source de gaz pilote, et dans laquelle les tiroirs respectifs (20) sont configurés afin d'ouvrir et de fermer de manière sélective les trajets d'écoulement de gaz (10f) dans le corps de vanne (10b) en réponse aux signaux impulsionnels de commande pilote transmis sur ces derniers afin de délivrer successivement ou simultanément de manière commandée une pluralité de gaz différents à partir de la vanne d'administration de gaz (10).

11. Vanne d'administration de gaz (10) selon la revendication 10, dans laquelle le corps de vanne comprend un orifice d'expiration à proximité de l'orifice de sortie (35), dans lequel la pluralité de réceptacles (10r) et de tiroirs (20) comporte un premier réceptacle (10r) et un premier tiroir correspondant (20A) disposés à proximité de l'orifice d'expiration et de l'orifice de sortie (35), un deuxième réceptacle (10r) et un deuxième tiroir (20B) espacés par rapport au premier tiroir (20A) et en communication fluidique avec ce dernier, le deuxième réceptacle (10r) présentant un orifice d'entrée de gaz B (31) afin de délivrer un gaz B non polarisé au cours du fonctionnement, et un troisième réceptacle (10r) et un troisième tiroir (20C) espacé par rapport au premier (20A) et au deuxième (20B) tiroirs et en communication fluidique avec le deuxième tiroir (20B), le troisième réceptacle (10r) présentant un orifice d'entrée de gaz A (33) afin de délivrer un gaz A hyperpolarisé au cours du fonctionnement, et dans laquelle l'orifice d'entrée de gaz vers le premier réceptacle (10r) est l'un ou l'autre des orifices de sortie (35, 235) à partir des deuxième et troisième réceptacles (10r) ou les deux.

12. Vanne d'administration de gaz (10) selon la revendication 10, dans laquelle les trajets d'écoulement de gaz (10f) comprennent un premier trajet d'écoulement de gaz d'inhalation, un deuxième trajet d'écoulement de gaz d'expiration, et un troisième trajet d'écoulement de gaz de retenue de respiration, chacun d'eux étant des trajets d'écoulement de gaz pouvant être sélectionnés à distance.

13. Vanne d'administration de gaz (10) selon la revendication 10, dans laquelle les tiroirs (20) et le corps de vanne (10b) sont agencés afin de permettre la fourniture de gaz hyperpolarisé, de gaz non polarisé, ou d'un mélange de gaz hyperpolarisé et de gaz non polarisé à partir des trajets d'écoulement de gaz d'inhalation et/ou de retenue de respiration (10f).

14. Vanne d'administration de gaz (10) selon la revendication 6, dans laquelle le tiroir (20) suit un mouvement alternatif entre des première et seconde positions opérationnelles (A1, A2), ladite vanne comprenant, en outre, deux électrovannes normalement fermées (V), une première associée de manière opérationnelle à chacun des orifices de fluide pilote (41, 43) et la source de gaz pilote, les électrovannes étant configurées, afin de commander l'activation du tiroir (20) entre les première et seconde positions, dans laquelle, en fonctionnement, après qu'un signal impulsionnel pilote de commande a été transmis à une tête de tiroir par l'intermédiaire de l'un sélectionné des orifices pilotes (41, 43) afin de forcer le tiroir (20) à se déplacer vers la seconde position (A2) à partir de la première position (A1), la vanne pilote normalement fermée respective (V) associée à l'orifice pilote sélectionné est mise à l'atmosphère de telle sorte que de la pression pilote dans la partie de réceptacle de tête de tiroir (10c) est réduite afin d'inhiber les interférences avec le signal de gaz pilote opposé à partir de l'autre orifice de pilote lorsque le tiroir de vanne est activé afin de renvoyer le tiroir (20) à sa première position (A1).

15. Vanne d'administration de gaz (10) selon la revendication 14, comprenant, en outre, un module formant ordinateur avec un code de programme informatique qui commande à distance la séquence de signaux de commande pilote vers les tiroirs (20) respectifs afin d'activer les tiroirs (20) et d'ouvrir et de fermer de manière automatique les trajets d'écoulement de gaz (10f) désirés dans le corps de vanne (10b) afin d'assurer la ventilation avec la séquence désirée de gaz hyperpolarisé et de gaz non polarisé.

16. Vanne d'administration de gaz (10) selon la revendication 11, comprenant, en outre, une pluralité de conduits s'étendant vers l'extérieur à partir du corps de vanne (10b) afin de définir une pluralité de trajets d'écoulement de gaz (10f), la pluralité de conduits comprenant deux conduits de trajet d'écoulement de gaz pilote pour chaque partie de réceptacle de tête de tiroir (10c) dans le corps de vanne (10b), au moins un conduit de gaz hyperpolarisé s'étendant à partir de la source de gaz hyperpolarisé vers un orifice d'entrée de gaz (33) correspondant dans le corps de vanne (10b), et au moins un conduit de gaz non polarisé s'étendant à partir d'une source de gaz non polarisé vers un orifice d'entrée de gaz (31) correspondant dans le corps de vanne (10b)
